(19) Europäisches Patentamt
European Patent Office
Office européen des brevets

(11) **EP 3 173 771 B1**

(12) **EUROPÄISCHE PATENTSCHRIFT**

(45) Veröffentlichungstag und Bekanntmachung des Hinweises auf die Patenterteilung:
**06.12.2023** **Patentblatt 2023/49**

(21) Anmeldenummer: **16196557.9**

(22) Anmeldetag: **31.10.2016**

(51) Internationale Patentklassifikation (IPC):
*G01N 21/78* (2006.01)    *A47L 15/42* (2006.01)
*G01N 33/18* (2006.01)    *D06F 34/22* (2020.01)
*G01N 21/80* (2006.01)    *G01N 21/77* (2006.01)
*D06F 39/08* (2006.01)    *D06F 103/20* (2020.01)
*D06F 105/42* (2020.01)    *D06F 105/58* (2020.01)
*D06F 105/60* (2020.01)

(52) Gemeinsame Patentklassifikation (CPC):
**G01N 21/78; A47L 15/4297; D06F 34/22;**
**G01N 33/1853;** D06F 39/088; D06F 2103/20;
D06F 2105/42; D06F 2105/58; D06F 2105/60;
G01N 21/80; G01N 2021/7763; G01N 2021/7793

(54) **WASSERFÜHRENDES HAUSHALTSGERÄT MIT EINER SENSORVORRICHTUNG ZUR BESTIMMUNG DER HÄRTE UND DES PH-WERTES EINER FLÜSSIGKEIT**

WATER-CARRYING HOUSEHOLD APPLIANCE WITH A SENSOR DEVICE FOR DETERMINING THE HARDNESS AND PH-VALUE OF A LIQUID

APPAREIL MÉNAGER CONTENANT DE L'EAU AVEC UN DISPOSITIF DE DÉTECTION POUR DÉTERMINER LA DURETÉ ET LA VALEUR PH D'UN LIQUIDE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **26.11.2015 DE 102015223371**

(43) Veröffentlichungstag der Anmeldung:
**31.05.2017 Patentblatt 2017/22**

(73) Patentinhaber: **BSH Hausgeräte GmbH**
**81739 München (DE)**

(72) Erfinder:
• **Fedorych, Oleh**
 **52062 Aachen (DE)**
• **Paintner, Kai**
 **86465 Welden (DE)**
• **Calvimontes, Alfredo**
 **89407 Dillingen a. d. Donau (DE)**

(56) Entgegenhaltungen:
DE-A1- 10 260 160    DE-A1-102009 029 305
DE-A1-102009 032 964    DE-A1-102012 011 195
DE-A1-102013 114 011    JP-A- 2001 201 497
KR-A- 20080 069 429    US-A1- 2007 178 595
US-B2- 6 567 166

**Beschreibung**

Die vorliegende Erfindung betrifft ein wasserführendes Haushaltsgerät mit einer Sensorvorrichtung zum Bestimmen der Wasserhärte und des pH-Wertes.

Herkömmliche wasserführende Haushaltsgeräte, wie eine Geschirrspülmaschine oder eine Waschmaschine, verwenden über die Hausanschlussleitung zugeführtes Leitungswasser. In diesem Leitungswasser befinden sich verschiedene gelöste Moleküle und/oder Ionen, wie Calciumionen und/oder Magnesiumionen. Üblicherweise wird in Abhängigkeit der Konzentration dieser Ionen von einer Wasserhärte gesprochen. Die Wasserhärte des Leitungswassers hängt von verschiedenen Faktoren, wie der Wasseraufbereitung sowie den geologischen Gegebenheiten, ab. Die Wasserhärte des Leitungswassers kann zeitlichen Schwankungen unterliegen und sich sogar innerhalb eines Tages verändern.

Die Wasserhärte hat einen negativen Einfluss auf wasserführende Haushaltsgeräte. Sie führt z.B. dazu, dass Waschmittel und/oder Spülmittel eine reduzierte Wirksamkeit aufweisen. Für ein zufriedenstellendes Ergebnis eines Waschvorgangs und/oder eines Spülvorgangs muss die Menge des Waschmittels und/oder des Spülmittels gemäß der Wasserhärte angepasst werden. Da jedoch häufig nur sehr grobe und veraltete Werte für die Wasserhärte des Leitungswassers vorliegen, muss in diesen Fällen von dem höchsten Wert innerhalb eines qualitativen Wertebereichs (weich, mittel, hart) ausgegangen werden, was zu einer Verschwendung von Waschmittel und/oder Spülmittel führen kann. Dies kann sich sowohl ökonomisch als auch ökologisch auswirken. Die Wasserhärte kann auch ein Verkalken von Haushaltsgeräten bewirken, was die Lebensdauer der Haushaltsgeräte stark reduzieren kann. Neben der Wasserhärte kann auch der pH-Wert des Leitungswassers eine wichtige Rolle für derartige Haushaltsgeräte spielen.

Es gibt technische Möglichkeiten, die Wasserhärte zu reduzieren, um so die negativen Effekte zu eliminieren. Ebenfalls der pH-Wert des Leitungswassers kann durch entsprechende Maßnahmen in einen gewünschten Bereich gebracht werden.

Dies setzt jedoch voraus, dass die Wasserhärte und/oder der pH-Wert des Leitungswassers bekannt sind.

Eine Möglichkeit zur Bestimmung der Wasserhärte ist eine spektrale Methode, wobei eine dünne Schicht in Abhängigkeit der Konzentration von Calciumionen und/oder Magnesiumionen ihre Farbe verändert. Dokument US 8,147,758 B2 beschreibt ein Beispiel einer entsprechenden Vorrichtung. Dokument US 3,652,861 A beschreibt eine weitere Vorrichtung sowie ein Verfahren zum Bestimmen der Wasserhärte. Dokument US 3,729,263 A zeigt eine weitere Vorrichtung zum Bestimmen der Wasserhärte mittels eines spektralen Verfahrens. Dokument DE 10 2012 011 195 A1 beschreibt eine weitere solche Vorrichtung. Dokument DE 10 2004 015 387 A1 zeigt eine weitere Alternative Vorrichtung zum Bestimmen der Wasserhärte. Weitere Analysegeräte, die für den Einsatz in der Prozessmesstechnik oder industriellen Messtechnik ausgelegt sind, sind aus US 2007/0178595 A1, DE 10 2009 029 305 A1 oder DE 2013 114 011 A1 bekannt.

JP 2001 201497 A beschreibt ein wasserführendes Haushaltsgerät (Boiler) mit einem Sensor zur Bestimmung der Wasserhärte umfassend zwei LEDs, einen ersten Behälter zur Aufnahme eines Gemisches aus Wasser und einem Indikator, einen Detektor, einen zweiten Behälter zur Aufnahme des Indikators und eine Zuführvorrichtung zum Zuführen des Indikators aus dem zweiten Behälter in den ersten Behälter. Die Wellenlängen der beiden LEDs werden dabei so ausgewählt, dass sowohl bei einer geringen als auch bei einer hohen Wasserhärte eine zuverlässige Wasserhärtebestimmung möglich ist.

Eine weitere Möglichkeit besteht darin, durch die Messung der Leitfähigkeit des Leitungswassers auf die im Leitungswasser enthaltenen Ionen zu schließen. Da hierbei jedoch jegliche Ionen die Leitfähigkeit beeinflussen, also auch solche, welche die Wasserhärte nicht betreffen, ist die Methode nicht genau.

Eine weitere Möglichkeit basiert auf einer Abhängigkeit der Transmission eines Lichtstrahls durch ein vorgegebenes Volumen eines Gemisches des Leitungswassers, welches eine unbekannte Konzentration von Calciumionen und/oder Magnesiumionen enthält, mit einem Farbindikator. Bei dieser Methode muss das vorgegebene Volumen des Leitungswassers manuell mit dem Indikator titriert werden. Darüber hinaus ist die Methode problembehaftet, da der Lichtstrahl bei der Transmission durch das Gemisch in Abhängigkeit der Wellenlänge unterschiedlich gebrochen wird. Dies führt dazu, dass aufwendige optische Vorrichtungen verwendet werden müssen, um eine Bestimmung zu ermöglichen.

Vor diesem Hintergrund besteht eine Aufgabe der vorliegenden Erfindung darin, die Wasserhärte und den pH-Wert des einem wasserführenden Haushaltsgerät zugeführten Wassers zu bestimmen.

Demgemäß wird ein wasserführendes Haushaltsgerät, insbesondere eine Geschirrspülmaschine, mit einer Sensorvorrichtung zum Bestimmen zumindest einer Eigenschaft einer Flüssigkeit entsprechend Anspruch 1 vorgeschlagen. Die Sensorvorrichtung umfasst:

- einen ersten Laser und einen zweiten Laser, wobei der erste Laser zum Abstrahlen eines ersten Lichtstrahls, welcher eine erste vorbestimmte Strahlcharakteristik mit einer ersten bestimmten Lichtintensität aufweist, eingerichtet ist, der zweite Laser zum Abstrahlen eines zweiten Lichtstrahls, welcher eine zweite vorbestimmte Strahlcharakteristik mit einer zweiten bestimmten Lichtintensität aufweist, eingerichtet ist,
- zumindest einen vor dem ersten und dem zweiten Laser angeordneten Behälter, der mit einem Gemisch aus der Flüssigkeit und zumindest einem Indikator befüllt ist,

- zumindest einen Detektor zum Detektieren einer durch den mit dem Gemisch befüllten Behälter transmittierten Lichtintensität des von dem ersten Laser abgestrahlten ersten Lichtstrahls und zum Detektieren einer durch den mit dem Gemisch befüllten Behälters transmittierten zweiten Lichtintensität des von dem zweiten Laser abgestrahlten zweiten Lichtstrahls, und zumindest einen weiteren Behälter zum Aufnehmen des zumindest einen Indikators und
- zumindest eine zwischen dem zumindest einen Behälter und dem zumindest einen weiteren Behälter angeordnete, steuerbare Zuführvorrichtung zum Zuführen des zumindest einen Indikators aus dem zumindest einen weiteren Behälter in den zumindest einen Behälter,

wobei der zumindest eine Indikator einen ersten und einen zweiten Indikator umfasst, und wobei die Sensorvorrichtung einen Wasserhärtesensor zum Bestimmen einer Härte der Flüssigkeit umfasst, wobei der erste Indikator dazu geeignet ist, mittels einer chemischen Reaktion mit in der Flüssigkeit enthaltenen Calciumionen und/oder Magnesiumionen ein lösliches Produkt zu formen, welches bei einer Emissionswellenlänge des ersten Lasers eine Absorption in Abhängigkeit der Konzentration der in der Flüssigkeit enthaltenen Calciumionen und/oder Magnesiumionen aufweist, jedoch keine Absorption bei einer Emissionswellenlänge des zweiten Lasers aufweist, und

die Sensorvorrichtung zusätzlich einen pH-Sensor zum Bestimmen eines pH-Wertes der Flüssigkeit umfasst, wobei der zweite Indikator dazu geeignet ist, mittels einer chemischen Reaktion in Abhängigkeit des pH-Wertes der Flüssigkeit ein lösliches Produkt zu formen, welches eine Absorption bei einer Emissionswellenlänge des zweiten Lasers, jedoch keine Absorption bei einer Emissionswellenlänge des ersten Lasers aufweist, und wobei die Sensorvorrichtung eine Bestimmungseinheit aufweist, welche dazu eingerichtet ist, die Härte und den pH-Wert der Flüssigkeit in Abhängigkeit von der einen detektierten Lichtintensität zu bestimmen.

Die Verwendung eines Lasers ermöglicht vorteilhafterweise, einen Lichtstrahl mit einer vorbestimmten Strahlcharakteristik auf einfache Weise zu erzeugen. Diese Strahlcharakteristik umfasst insbesondere eine sehr schmale Linienbreite, eine sehr gute Kollimation, eine hohe Intensität, ein bekanntes Strahlprofil und/oder eine Polarisation des Lichtstrahls. Die Linienbreite gibt hierbei an, wie die Intensität des Lichtstrahls von der Wellenlänge abhängt. Die Kollimation gibt an, wie groß die Aufweitung des Lichtstrahls über eine vorgegebene Distanz ist. Die Intensität gibt an, wieviel Energie in dem Lichtstrahl transportiert wird. insbesondere kann ein Lichtstrahl mit einer hohen Intensität auch nach einer sehr starken Abschwächung, z.B. durch Absorption, noch mit einfachen technischen Mitteln nachgewiesen werden. Das Strahlprofil gibt an, wie sich die Intensität innerhalb des Lichtstrahls auf einer Achse orthogonal zur Ausbreitungsrichtung des Lichtstrahls verändert. Die Polarisation gibt an, ob das elektrische Feld bei der Propagation des Lichtstrahls eine Vorzugsrichtung besitzt.

Die Bestimmungseinheit kann hardwaretechnisch und/oder auch softwaretechnisch implementiert sein. Bei einer hardwaretechnischen Implementierung kann die Bestimmungseinheit als Vorrichtung oder als Teil einer Vorrichtung, zum Beispiel als Computer oder als Mikroprozessor ausgebildet sein. Bei einer softwaretechnischen Implementierung kann die Bestimmungseinheit als Computerprogrammprodukt, als eine Funktion, als eine Routine, als Teil eines Programmcodes oder als ausführbares Objekt ausgebildet sein. Dies ermöglicht vorteilhafterweise eine einfache Reprogrammierung der für den Betrieb der Sensorvorrichtung benötigten Betriebsdaten.

Gemäß einer weiteren Ausführungsform des wasserführenden Haushaltsgeräts umfasst die Sensorvorrichtung einen Detektor, dessen Detektionsfläche derart dimensioniert ist, dass der gesamte Strahlquerschnitt des zumindest einen Lichtstrahls durch den Detektor detektierbar ist.

Dies ermöglicht vorteilhafterweise eine einfache Detektion der gesamten durch den Behälter transmittierten Lichtintensität. Dies erlaubt insbesondere eine genaue Bestimmung der zumindest einen Eigenschaft der Flüssigkeit durch die Bestimmungseinheit.

Gemäß einer weiteren Ausführungsform des wasserführenden Haushaltsgeräts ist der Behälter der Sensorvorrichtung als ein Kunststoffbehälter oder ein Glasbehälter ausgebildet, wobei zumindest zwei Seitenwände des Behälters in zu dem zumindest einen Lichtstrahl normaler Richtung planparallel ausgeführt sind und in einem Bereich einer Emissionswellenlänge des zumindest einen Lasers transparent sind.

Transparent bedeutet insbesondere, dass die Lichtintensität beim Durchstrahlen einer Seitenwand nicht so stark abgeschwächt wird, dass die durch den leeren Behälter transmittierte und vom Detektor detektierte Lichtintensität null ist. Anders ausgedrückt bedeutet transparent, dass die durch den leeren Behälter transmittierte Lichtintensität noch so groß ist, dass diese vom Detektor detektiert werden kann.

Die Wahl des Kunststoffs oder Glases für den Behälter erfolgt vorteilhafterweise so, dass der Behälter verschiedene produktionsbedingte, einsatzbedingte und/oder weitere wunschgemäße Anforderungen erfüllt. Diese Anforderungen können z.B. durch eine Ausformung in einem Spritzgussverfahren bedingt sein, benötigte Resistenz gegen gewisse Chemikalien oder auch ein besonders leichtes und/oder kostengünstiges Material sein.

Die planparallele Ausrichtung in zu dem einen Lichtstrahl normaler Richtung eliminiert vorteilhafterweise die Brechung des Lichtstrahls bei der Transmission durch den Behälter. insbesondere ist damit auch eine eventuelle Dispersion von mehreren Lichtstrahlen unterschiedlicher Wellenlänge verhindert. Unter Dispersion wird hierbei verstanden, dass Lichtstrahlen unterschiedlicher Wellenlänge unterschiedlich stark gebrochen werden.

Gemäß einer weiteren Ausführungsform des wasserführenden Haushaltsgeräts umfasst der zumindest eine Behälter der Sensorvorrichtung eine Mischvorrichtung zum Durchmischen des Gemisches.

Damit ist vorteilhafterweise sichergestellt, dass das Gemisch aus der Flüssigkeit und dem zumindest einen Indikator optimal durchmischt ist. Eine optimale Durchmischung gewährleistet insbesondere eine homogene Verteilung des zumindest einen Indikators in der Flüssigkeit. Somit ist ermöglicht, dass eine chemische Reaktion zwischen in der Flüssigkeit gelösten Substanzen und dem zumindest einen Indikator quantitativ abläuft. Quantitativ bedeutet, dass alle vorhanden Moleküle und/oder Ionen einer vorgegebenen Sorte bei der Reaktion umgesetzt werden.

Gemäß einer weiteren Ausführungsform des wasserführenden Haushaltsgeräts umfasst der zumindest eine Detektor der Sensorvorrichtung eine Fotodiode, wobei die Fotodiode eine Empfindlichkeit bei einer Emissionswellenlänge des zumindest einen Lasers aufweist.

Die Verwendung einer Fotodiode ermöglicht vorteilhafterweise eine einfache und leicht integrierbare Realisierung des Detektors.

Die Sensorvorrichtung umfasst zumindest einen weiteren Behälter zum Aufnehmen des zumindest einen Indikators, sowie zumindest eine zwischen dem zumindest einen Behälter und dem zumindest einen weiteren Behälter angeordnete, steuerbare Zuführvorrichtung zum Zuführen des zumindest einen Indikators aus dem zumindest einen weiteren Behälter in den zumindest einen Behälter.

Durch die Integration eines weiteren Behälters für den zumindest einen Indikator kann vorteilhafterweise eine Menge des Indikators vorgehalten werden, so dass eine Anzahl von Messungen durchgeführt werden kann, ohne dass für jede Messung der Indikator von Hand zugeführt werden muss. Eine Anzahl kann hierbei z.B. 100 oder auch 1000 bedeuten. Eine Messung bedeutet, dass die zumindest eine Eigenschaft der Flüssigkeit durch die Sensorvorrichtung ermittelt wird. Somit wird für den Benutzer des Haushaltsgerätes erreicht, dass dieser den Indikator nicht manuell zuführen muss, was das Risiko des Kontaktes mit dem potenziell gefährlichen Indikator eliminiert.

Ein Vorteil der steuerbaren Zuführvorrichtung besteht darin, dass diese automatisch, wenn es benötigt wird, den Indikator in den Behälter zudosiert. Die steuerbare Zuführvorrichtung ist insbesondere ein steuerbares Ventil. Unter Zudosieren wird hier verstanden, dass die zugeführte Menge des Indikators einem vorgegebenen Anteil des Gemisches entspricht. Der vorgegebene Anteil kann beispielsweise 10% bezogen auf ein Volumen, eine Stoffmenge und/oder ein Gewicht des Gemisches sein. Dies ist vorteilhaft für die korrekte Ermittlung der zumindest einen Eigenschaft der Flüssigkeit. Das Risiko einer fehlerhaften Messung durch eine falsche Zudosierung des Indikators ist damit eliminiert.

Die Sensorvorrichtung ist als ein Wasserhärtesensor zum Bestimmen einer Härte der Flüssigkeit ausgebildet, wobei der Indikator dazu geeignet ist, mittels einer chemischen Reaktion mit in der Flüssigkeit enthaltenen Calciumionen und/oder Magnesiumionen ein lösliches Produkt zu formen, welches bei einer Emissionswellenlänge des zumindest einen Lasers eine Absorption in Abhängigkeit der Konzentration der in der Flüssigkeit enthaltenen Calciumionen und/oder Magnesiumionen aufweist.

Die Ausführung der Sensorvorrichtung als Wasserhärtesensor ist aufgrund des verwendeten Lasers besonders vorteilhaft. Durch die Verwendung eines Lasers kann eine sehr hohe Intensität des Lichtstrahls erreicht werden. Daher kann eine transmittierte Lichtintensität des Lichtstrahls nach dem mit dem Gemisch gefüllten Behälter auch bei hoher Absorption des Lichtstrahls durch das Gemisch noch nachgewiesen werden. Dies ermöglicht vorteilhafterweise die Verwendung einer großen Menge an Indikator. Die große Menge bedeutet hierbei, dass diese ausreicht, um einen quantitativen Umsatz der in der Flüssigkeit enthaltenen Calciumionen und/oder Magnesiumionen durch die chemische Reaktion zu erzielen.

Die chemische Reaktion ist z.B. eine Komplexbildung. Derartige Komplexe haben häufig Absorptionsbanden im sichtbaren Lichtspektrum oder auch im ultravioletten Spektralbereich oder infraroten Spektralbereich. Daher absorbieren sie Lichtstrahlen mit einer entsprechenden Wellenlänge. Der Indikator und/oder der Laser wird erfindungsgemäß so ausgewählt, dass das geformte lösliche Produkt eine Absorption im Bereich der Emissionswellenlänge des Lasers besitzt.

Zusätzlich ist die Sensorvorrichtung als pH-Sensor zum Bestimmen eines pH-Wertes der Flüssigkeit ausgebildet. Dann ist der Indikator dazu geeignet, mittels einer chemischen Reaktion in Abhängigkeit des pH-Wertes der Flüssigkeit ein lösliches Produkt zu formen, welches bei einer Emissionswellenlänge des zumindest einen Lasers eine Absorption aufweist.

Der pH-Wert der Flüssigkeit ist dabei durch eine Funktion der Wasserstoffionenkonzentration in der Flüssigkeit gegeben.

Erfindungsgemäß umfasst die Sensorvorrichtung einen Wasserhärtesensor zum Bestimmen einer Härte der Flüssigkeit und einen pH-Sensor zum Bestimmen eines pH-Wertes der Flüssigkeit.

Gemäß einer weiteren Ausführungsform des wasserführenden Haushaltsgeräts umfasst die Sensorvorrichtung einen einzigen vor dem ersten Laser und dem zweiten Laser angeordneten Behälter zum Aufnehmen des Gemisches, und einen einzigen Detektor zum Detektieren einer durch den mit dem Gemisch befüllten Behälter transmittierten ersten Lichtintensität des von dem ersten Laser abgestrahlten ersten Lichtstrahls und zum Detektieren einer durch den mit dem Gemisch befüllten Behälters transmittierten zweiten Lichtintensität des von dem zweiten Laser abgestrahlten zweiten Lichtstrahls.

Dabei ist die Verwendung genau eines Behälters und genau eines Detektors vorteilhaft, da der Aufbau der Sensorvorrichtung damit vereinfacht wird. Darüber hinaus können Kosten eingespart werden, da weniger Komponenten eingesetzt werden müssen, als wenn für jede der zumindest einen Eigenschaft der Flüssigkeit separate Behälter und Detektoren eingesetzt werden.

Gemäß einer weiteren Ausführungsform des wasserführenden Haushaltsgeräts umfasst die Sensorvorrichtung eine Steuerungseinrichtung, welche dazu eingerichtet ist, das Füllen des Behälters mit der Flüssigkeit, das Zuführen eines vorgegebenen Anteils des zumindest einen Indikators über die zumindest eine Zuführvorrichtung von dem zumindest einen weiteren Behälter in den Behälter, das Durchmischen des Gemisches durch die Mischvorrichtung, das Abstrahlen des Lichtstrahls durch den zumindest einen Laser, das Detektieren der durch den Behälter transmittierten Lichtintensität des zumindest einen Lichtstrahls durch den Detektor, das Bestimmen der zumindest einen Eigenschaft der Flüssigkeit in Abhängigkeit von der zumindest einen transmittierten Lichtintensität durch die Bestimmungseinheit, ein Speichern der zumindest einen Eigenschaft der Flüssigkeit auf einem Speichermedium und/oder ein Ausgeben der zumindest einen Eigenschaft der Flüssigkeit über eine Ausgabevorrichtung zu veranlassen.

Die Steuerungseinrichtung ermöglicht damit vorteilhafterweise einen vollautomatischen Betrieb der Sensorvorrichtung. Die Steuerungseinrichtung kann dabei vorteilhafterweise sowohl für den Betrieb der Sensorvorrichtung als auch den Betrieb des Haushaltsgeräts verwendet werden.

Die Steuerungseinrichtung kann beispielsweise auch eine Eingabevorrichtung und Eingabemittel umfassen, womit die Sensorvorrichtung von einem Benutzer gesteuert werden kann. Die Eingabemittel können z. B. über Tasten an dem Haushaltsgerät realisiert sein oder auch durch einen berührungsempfindlichen Bildschirm.

Die Ausgabevorrichtung umfasst z.B. ein Display, insbesondere einen berührungsempfindlichen Bildschirm, eine drahtgebundene und/oder drahtlose Datenverbindung, und/oder einen Lautsprecher. Über eine Datenverbindung kann die zumindest eine Eigenschaft der Flüssigkeit auf ein externes Gerät, z.B. ein Personal Computer, Laptop, PDA und/oder Smartphone übertragen werden. Dies ist besonders benutzerfreundlich, da ein Benutzer somit nicht das Haushaltsgerät aufsuchen muss, um die gewünschten Informationen zu erhalten. Insbesondere können hierbei auch entsprechende Informationen von mehreren Haushaltsgeräten an einer zentralen Einheit abgerufen werden. Zudem kann die Datenverbindung dazu verwendet werden, weitere aktuelle Statusangaben über das Haushaltsgerät zu übertragen, wie z.B. verbleibende Zeit bis zu einem Programmende eines Geschirrspülprogramms einer Geschirrspülmaschine, eventuell vorliegende Wartungs- und/oder Fehlerinformationen, insbesondere auch der Füllstand des Indikatorbehälters und dergleichen mehr. Dies hat den weiteren Vorteil, dass ein Benutzer oder auch Servicetechniker komfortabel an einer zentralen Einheit alle Informationen zu dem Haushaltsgerät betrachten und analysieren kann. Zudem kann, beispielsweise wenn der Indikator aufgebraucht ist, über eine externe Datenverbindung, wie eine Internetverbindung, eine automatische Nachbestellung oder ein entsprechender Serviceauftrag an den Hersteller ergehen. Diese Funktionalität kann auch bei anderen Problemen mit dem Haushaltsgerät eingesetzt werden. Weiterhin können mittels der Datenverbindung weitere Eingabemittel, die beispielsweise von einem externen Gerät bereitgestellt werden, eingebunden werden. Somit kann die Steuerungseinrichtung auch von dem externen Gerät gesteuert werden.

Das wasserführende Haushaltsgerät ist insbesondere eine Geschirrspülmaschine, eine Waschmaschine, ein Wäschetrockner, eine Kaffeemaschine, ein Wasserkocher und/oder ein Dampfgarer.

Weitere mögliche Implementierungen der Erfindung umfassen auch nicht explizit genannte Kombinationen von zuvor oder im Folgenden bezüglich der Ausführungsbeispiele beschriebenen Merkmale oder Ausführungsformen.

Weitere vorteilhafte Ausgestaltungen und Aspekte der Erfindung sind Gegenstand der Unteransprüche sowie der im Folgenden beschriebenen Ausführungsbeispiele der Erfindung. Im Weiteren wird die Erfindung anhand von bevorzugten Ausführungsformen unter Bezugnahme auf die beigelegten Figuren näher erläutert.

Fig. 1 zeigt ein schematisches Blockschaltbild einer Sensorvorrichtung mit einem Laser;

Fig. 2 zeigt ein Schema der Lichtintensität vor und nach dem Behälter der Sensorvorrichtung der Fig. 1;

Fig. 3 zeigt eine Draufsicht auf den Detektor der Sensorvorrichtung der Fig. 1 in Richtung eines Lichtstrahls;

Fig. 4 zeigt ein schematisches Blockschaltbild einer ersten Ausführungsform der Sensorvorrichtung;

Fig. 5 zeigt ein schematisches Blockschaltbild einer zweiten Ausführungsform der Sensorvorrichtung;

Fig. 6 zeigt ein schematisches Blockschaltbild eines Gehäuses umfassend eine Sensorvorrichtung nach einer der Fig. 1, 4 oder 5;

Fig. 7 zeigt ein schematisches Blockschaltbild eines wasserführenden Haushaltsgeräts mit einer Sensorvorrichtung nach einer der Fig. 1, 4 oder 5; und

Fig. 8 zeigt ein Verfahren zum Betreiben der Sensorvorrichtung.

In den Figuren sind gleiche oder funktionsgleiche Elemente mit denselben Bezugzeichen versehen worden, sofern nichts anderes angegeben ist.

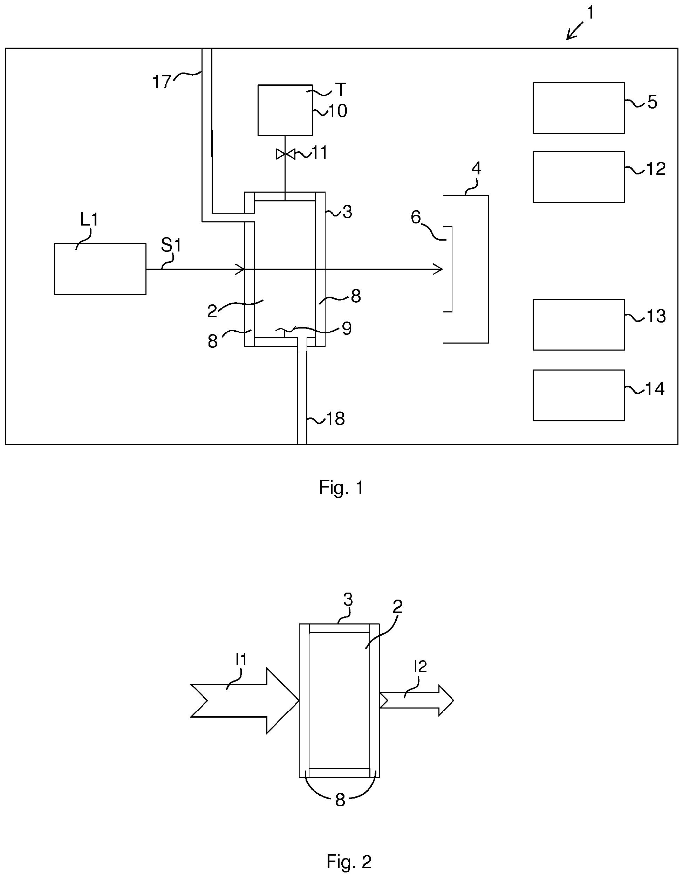

Fig. 1 zeigt ein schematisches Blockschaltbild der Sensorvorrichtung 1. In dem Beispiel der Fig. 1 ist die Sensorvorrichtung 1 als ein Wasserhärtesensor oder ein pH-Sensor für Leitungswasser ausgebildet.

Die Sensorvorrichtung 1 weist einen Laser L1, einen Behälter 3 zur Aufnahme eines Gemisches 2 aus zugeführtem Leitungswasser und einem Indikator T sowie einen Detektor 4 mit einer Detektionsfläche 6 auf.

Der Laser L1 ist dazu geeignet, einen Lichtstrahl S1 abzustrahlen, wobei der Lichtstrahl S1 eine vorbestimmte Strahlcharakteristik mit einer bestimmten Lichtintensität I1 (siehe Fig. 2) aufweist. Die Richtung des Lichtstrahls S1 ist dabei durch Pfeile angedeutet.

Der Behälter 3 weist insbesondere zwei transparente und planparallele Seitenwände 8 auf. Ferner umfasst der Behälter 3 eine Mischungsvorrichtung 9 zum Durchmischen des enthaltenen Gemisches 2.

Die Sensorvorrichtung 1 umfasst eine Leitungswasserzuführung 17, welche den Behälter 3 mit einem nicht näher gezeigten, externen Wassernetz verbindet und welche dazu eingerichtet ist, den Behälter 3 mit Leitungswasser zu füllen, sowie eine Auslassleitung 18, welche dazu eingerichtet ist, nach einer Messung das Gemisch 2 aus dem Behälter 3 abzuleiten.

Insbesondere ist die Leitungswasserzuführung 17 zusammen mit der Auslassleitung 18 dazu geeignet, den Behälter 3 mit Leitungswasser zu spülen. Damit werden Rückstände des Gemisches 2 aus dem Behälter 3 entfernt und somit eine Verfälschung von Messungen durch eine Kontamination verhindert. Das Spülen kann insbesondere nach einer Messung und/oder vor einer Messung erfolgen.

Im Beispiel der Fig. 1 ist der Indikator T in einem weiteren Behälter 10 enthalten, wobei der weitere Behälter 10 mittels einer Zuführvorrichtung 11 mit dem Behälter 3 verbunden ist.

Des Weiteren umfasst die Sensorvorrichtung 1 der Fig. 1 eine Bestimmungseinheit 5 zum Bestimmen der Wasserhärte und/oder des pH-Wertes des Leitungswassers. Die Bestimmungseinheit 5 verwendet dabei die vom Detektor 4 detektierte transmittierte Lichtintensität I2 (siehe Fig. 2) des Lichtstrahls S1, um die Wasserhärte und/oder den pH-Wert des Leitungswassers zu bestimmen.

Der Detektor 4 ist dazu eingerichtet, eine transmittierte Lichtintensität I2 zu detektieren und diese an die Bestimmungseinheit 5 auszugeben. Die Detektionsfläche 6 ist dabei beispielsweise als CCD, als CMOS oder als ein weiteres lichtempfindliches Bauteil, insbesondere als Fotodiode, ausgeführt.

Weiterhin umfasst die Sensorvorrichtung 1 der Fig. 1 eine Steuerungseinrichtung 12, ein Speichermedium 13 und eine Ausgabevorrichtung 14. Die Steuerungseinrichtung 12 ist dazu eingerichtet, die weiteren Komponenten der Sensorvorrichtung 1 anzusteuern. Dieses Ansteuern erfolgt insbesondere derart, dass die Wasserhärte und/oder der pH-Wert des Leitungswassers bestimmt werden. Das Ansteuern umfasst dabei ein Anschalten des Lasers L1 zum Abstrahlen des Lichtstrahls S1 mit der vorbestimmten Strahlcharakteristik und der bestimmten Lichtintensität I1, ein Befüllen des Behälters 3 mit Leitungswasser mittels der Leitungswasserzuführung 17, ein Zuführen des Indikators T mittels der Zuführvorrichtung 11, ein Durchmischen des Gemisches 2 in dem Behälter 3 mittels der Mischvorrichtung 9, ein Detektieren der transmittierten Lichtintensität I2 mittels des Detektors 4, ein Bestimmen der Wasserhärte und/oder des pH-Wertes mittels der Bestimmungseinheit 5 sowie ein Speichern der Wasserhärte und/oder des pH-Wertes mittels des Speichermediums 13 und/oder ein Ausgeben der Wasserhärte und/oder des pH-Wertes mittels der Ausgabevorrichtung 14.

Die Steuerungseinrichtung 12 kann hardwaretechnisch und/oder auch softwaretechnisch implementiert sein. Bei einer hardwaretechnischen Implementierung kann die Steuerungseinrichtung 12 als Vorrichtung oder als Teil einer Vorrichtung, zum Beispiel als Computer oder als Mikroprozessor ausgebildet sein. Bei einer softwaretechnischen Implementierung kann die Bestimmungseinheit als Computerprogrammprodukt, als eine Funktion, als eine Routine, als Teil eines Programmcodes oder als ausführbares Objekt ausgebildet sein.

Das Speichermedium 13 ist bevorzugt als permanenter Massenspeicher, beispielsweise als Magnetspeicher oder als Flashspeicher, ausgeführt und ist dazu geeignet, eine Mehrzahl an Messwerten dauerhaft abzuspeichern, auf Abruf bereitzustellen und auch wieder zu löschen. Ein Messwert bezeichnet dabei beispielsweise das Ergebnis der Bestimmung der Wasserhärte und/oder des pH-Wertes des Leitungswassers.

Die Ausgabevorrichtung 14 ist dazu eingerichtet, einen Messwert auszugeben. Dies kann z.B. durch eine akustische Ausgabe stattfinden, wobei die Ausgabevorrichtung 14 dann einen Lautsprecher umfasst. Die Ausgabe kann auch mittels einer Anzeige des Messwertes auf einem Bildschirm, insbesondere einem berührungsempfindlichen Bildschirm, oder auf einer alternativen Anzeige, beispielsweise einer Digitalanzeige oder einem Zeigermessinstrument, erfolgen.

Insbesondere umfasst die Ausgabevorrichtung 14 dabei auch Ausgabemittel, um Daten, wie Messwerte, zu übertragen. Die Ausgabemittel sind dabei beispielsweise als kabelgebundene oder kabellose Schnittstelle ausgeführt. Kabelgebundene Schnittstellen sind beispielsweise USB, Firewire, LAN, RS-232. Kabellose Schnittstellen umfassen WLAN, Bluetooth®, Funk, RFID.

Fig. 2 zeigt ein Schema der Abschwächung des Lichtstrahls S1, S2 von einer bestimmten Lichtintensität I1 vor dem Behälter 3 auf eine transmittierte Lichtintensität I2 nach der Transmission des Lichtstrahls S1, S2 durch den mit dem Gemisch 2 befüllten Behälter 3.

In dem Gemisch 2 läuft zwischen der Flüssigkeit und dem Indikator T eine chemische Reaktion ab. Die chemische Reaktion kann dabei z.B. durch folgende Reaktionsgleichung beschrieben werden:

A + B(Überschuss) → AB + B(Überschuss)

Hierbei steht A für die in der Flüssigkeit enthaltenen Calciumionen und/oder Magnesiumionen, B steht für den Indikator T, der im Überschuss vorliegt und AB ist das aufgrund der chemischen Reaktion zwischen den in der Flüssigkeit enthaltenen Calciumionen und/oder Magnesiumionen mit dem Indikator T geformten löslichen Produkts.

Der Indikator T kann eine Flüssigkeit oder auch ein Feststoff sein. Wenn der Indikator T ein Feststoff ist, dann wird vor einer Messung sichergestellt, dass sich der gesamte zugeführte Feststoff aufgelöst hat. Dies kann beispielsweise mittels der Mischvorrichtung 9 geschehen, indem diese für eine bestimmte Zeitdauer nach der Zudosierung des Indikators T und vor einer Messung aktiviert wird. Die bestimmte Zeitdauer umfasst hierbei eine Zeitdauer, nach welcher der Lösungsprozess des Indikators T in der Flüssigkeit abgeschlossen ist.

Die durch den mit dem Gemisch 2 gefüllten Behälter 3 transmittierte Lichtintensität I2 des Lichtstrahls S1, S2 kann mithilfe des Lambert-Beer'schen Gesetzes beschrieben werden:

$$I2 = I1 * \exp(-k * c * d).$$

Hierbei ist k Extinktionskoeffizient des löslichen Produktes, c ist die Konzentration des löslichen Produktes und d ist die Wegstrecke, die der Lichtstrahl S1, S2 in dem Gemisch 2 zurücklegt. Da k sowie d konstant und bekannt sind, und die bestimmte Lichtintensität I1 ebenfalls ermittelt werden kann, kann durch Auflösen der Gleichung nach c die Konzentration des löslichen Produktes berechnet werden.

Vorzugsweise wird zunächst eine Messung der transmittierten Lichtintensität I2 durchgeführt, wobei der Behälter 3 ausschließlich mit der Flüssigkeit gefüllt ist. Die somit ermittelte Lichtintensität I2 wird anschließend von der Bestimmungseinheit 5 als Referenzintensität für eine folgende weitere Messung verwendet. Die weitere Messung wird durchgeführt, sobald der Flüssigkeit in dem Behälter 3 die bestimmte Menge des Indikators T mittels der Zuführvorrichtung 11 zugeführt wurde und das Gemisch 2 mittels der Mischvorrichtung 9 durchmischt wurde. Aus einem Vergleich der zuerst ermittelten Referenzintensität mit der dann ermittelten transmittierten Lichtintensität I2 kann die Absorption durch das Gemisch 2 festgestellt werden. Aus der Absorption lässt sich unter Verwendung des Lambert-Beer'schen Gesetzes (siehe oben) die Konzentration des löslichen Produktes ermitteln. Diese Ausführung ist besonders vorteilhaft, da hiermit Einflüsse auf die transmittierte Lichtintensität I2, die beispielsweise von dem Behälter 3 kommen, nicht gesondert beachtet werden müssen.

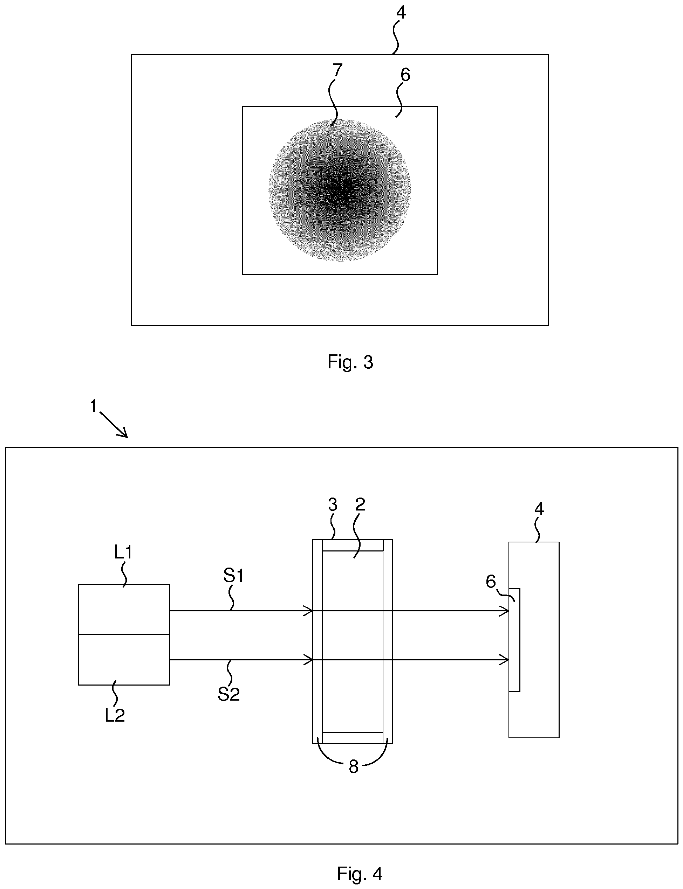

Fig. 3 zeigt eine Draufsicht auf den Detektor 4 in Richtung eines Lichtstrahls S1, S2. In dem Beispiel der Fig. 3 sind zudem die Detektionsfläche 6 sowie ein Strahlquerschnitt 7 des Lichtstrahls S1, S2 gezeigt.

In der in Fig. 3 dargestellten Ausführungsform ist die Detektionsfläche 6 des Detektors 4 so dimensioniert, dass der gesamte Strahlquerschnitt 7 des Lichtstrahls S1, S2 innerhalb der Detektionsfläche 6 liegt.

Fig. 4 zeigt ein schematisches Blockschaltbild einer ersten Ausführungsform der Sensorvorrichtung 1. Die erste Ausführungsform der Fig. 4 umfasst alle Merkmale der Sensorvorrichtung nach Fig. 1, welche aus Gründen der Übersichtlichkeit nicht alle erneut dargestellt werden. Die erste Ausführungsform umfasst einen ersten Laser L1 zum Abstrahlen eines ersten Lichtstrahls S1 sowie einen zweiten Laser L2 zum Abstrahlen eines zweiten Lichtstrahls S2.

Gemäß der Fig. 4 ist der Behälter 3 mit einem Gemisch 2 befüllt, wobei das Gemisch 2 eine Flüssigkeit, einen ersten Indikator T sowie einen zweiten Indikator T umfasst. Der erste Indikator T bildet mittels einer chemischen Reaktion in Abhängigkeit einer ersten Eigenschaft, insbesondere einer Wasserhärte, der Flüssigkeit ein lösliches Produkt, wobei das lösliche Produkt eine Absorption in Abhängigkeit der ersten Eigenschaft im Bereich der Emissionswellenlänge des ersten Lasers L1, jedoch keine Absorption im Bereich der Emissionswellenlänge des zweiten Lasers L2 aufweist. Der zweite Indikator T bildet mittels einer chemischen Reaktion in Abhängigkeit einer zweiten Eigenschaft, insbesondere einem pH-Wert, der Flüssigkeit ein lösliches Produkt, wobei das lösliche Produkt eine Absorption in Abhängigkeit der zweiten Eigenschaft im Bereich der Emissionswellenlänge des zweiten Lasers L2, jedoch keine Absorption im Bereich der Emissionswellenlänge des ersten Lasers L1 aufweist. Darüber hinaus ist umfasst der Behälter 3 zwei planparallele Seitenwände 8, welche in dem jeweiligen Bereich der Emissionswellenlänge des ersten Lasers L1 sowie des zweiten Lasers L2 transparent sind.

Weiterhin ist im Beispiel der Fig.4 die Detektionsfläche 6 des Detektors 4 derart ausgebildet, dass sowohl der erste Lichtstrahl S1 als auch der zweite Lichtstrahl S2 auf die Detektionsfläche 6 treffen und somit vom Detektor 4 detektiert werden können.

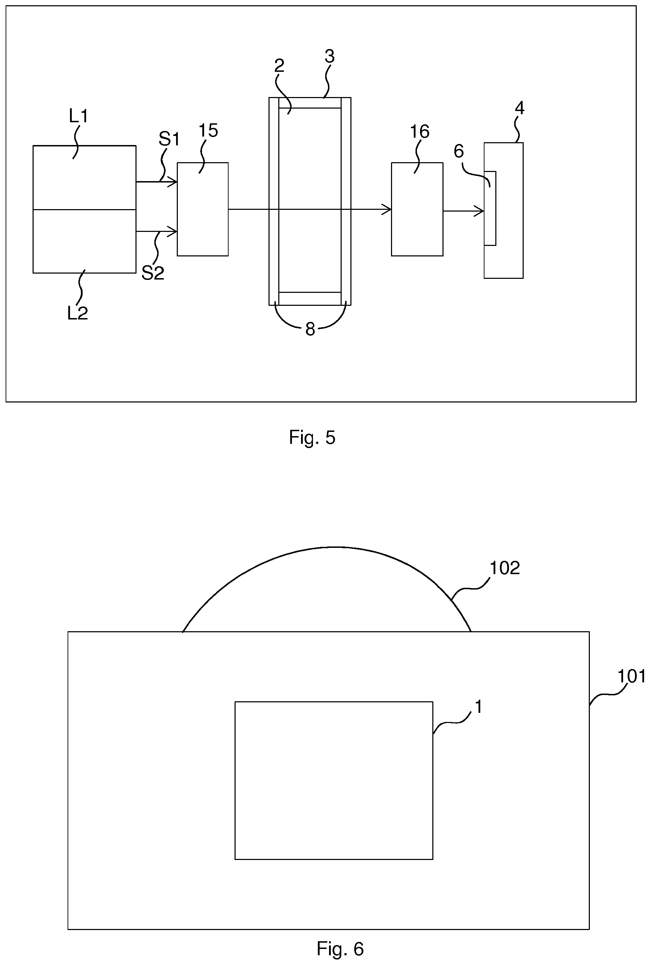

Fig. 5 zeigt ein schematisches Blockschaltbild einer zweiten Ausführungsform der Sensorvorrichtung 1. Die zweite Ausführungsform der Fig. 5 umfasst alle Merkmale der Sensorvorrichtung nach Fig. 1, welche aus Gründen der Übersichtlichkeit nicht alle erneut dargestellt werden. Gemäß der Fig. 5 umfasst die Sensorvorrichtung 1 einen ersten Laser L1 zur Abstrahlung eines ersten Lichtstrahls S1, einen zweiten Laser L2 zur Abstrahlung eines zweiten Lichtstrahls S2, einen Behälter 3 zur Aufnahme eines Gemisches 2 und einen Detektor 4 mit einer Detektionsfläche 6. Darüber hinaus umfasst die Sensorvorrichtung 1 in dieser zweiten Ausführungsform eine Zusammenführungsoptik 15 sowie eine Fokussieroptik 16.

Die Zusammenführungsoptik 15 ist dazu eingerichtet, die Lichtstrahlen S1, S2 so umzulenken, dass sie am Ausgang der Zusammenführungsoptik 15 aufeinander fallen und sich kollinear ausbreiten. Dies hat den Vorteil, dass jeder der zwei Lichtstrahlen S1, S2 den gleichen optischen Weg geht. Die Zusammenführungsoptik 15 kann hierbei beispielsweise ein System von Spiegeln und/oder Linsen umfassen. Es ist auch denkbar, dass mehr als zwei Laser L1, L2 verwendet werden (nicht dargestellt). Dann ist die Zusammenführungsoptik 15 dazu eingerichtet, alle der mehr als zwei Lichtstrahlen S1, S2, die von den mehr als zwei Lasern L1, L2 abgestrahlt werden, so umzulenken, dass sie am Ausgang der Zusammenführungsoptik 15 aufeinander fallen und sich kollinear ausbreiten.

Der Behälter 3 in der Fig. 5 umfasst insbesondere zwei planparallele Seitenwände 8, welche in dem jeweiligen Bereich der Emissionswellenlänge der mehreren Laser L1, L2 transparent sind.

Die Fokussieroptik 16 ist dazu eingerichtet, die Lichtstrahlen S1, S2, nachdem diese den Behälter 3 mit dem darin enthaltenen Gemisch 2 passiert haben, wobei die Lichtstrahlen S1, S2 im gezeigten Ausführungsbeispiel aufeinander liegen, auf die Detektionsfläche 6 des Detektors 4 zu fokussieren. Dies hat den besonderen Vorteil, dass die Detektionsfläche 6 kleiner ausgeformt werden kann, was Kosten bei der Herstellung des Detektors 4 einsparen kann. Des Weiteren bietet diese Ausführung den Vorteil, dass durch die Fokussieroptik 16 ein sich eventuell aufweitender Lichtstrahl S1, S2 aufgefangen wird. Darüber hinaus kann die Detektionsgrenze durch ein Fokussieren der Lichtstrahlen S1, S2 gesenkt werden. Unter der Detektionsgrenze wird hierbei die geringste durch den Detektor 4 nachweisbare transmittierte Lichtintensität I2 verstanden. Die Detektionsgrenze hängt unter anderem vom Aufbau des Detektors 4, insbesondere von der Beschaffenheit der Detektionsfläche 6, ab. Die Fokussieroptik 16 umfasst beispielsweise eine Anordnung von Spiegeln und/oder Linsen.

Im Beispiel der Fig. 5 ist der Detektor 4 dazu eingerichtet, die transmittierte Intensität I2 der beiden Lichtstrahlen S1, S2 gleichzeitig zu detektieren und getrennt voneinander als Detektionssignale auszugeben.

Alternativ dazu kann auch entweder die Zusammenführungsoptik 15 oder die Fokussieroptik 16 ein Bauteil enthalten, welches nur einen der beiden Lichtstrahlen S1, S2 passieren lässt. Dann wird zunächst nur der erste Lichtstrahl S1 durchgelassen, um eine Messung durchzuführen, und danach wird nur der zweite Lichtstrahl S2 durchgelassen um eine weitere Messung durchzuführen. Das Bauteil kann beispielsweise als umschaltbarer Spiegel, als spektrale Filtereinheit und/oder als Shutter ausgeführt sein. Damit wird erreicht, dass die Messung mittels mehrerer Laser L1, L2 zeitlich nacheinander erfolgen kann. Anstelle des separaten Bauteils ist es auch denkbar, dass durch die Steuerungseinrichtung 12 zunächst nur der erste Laser L1 zum Ausgeben eines ersten Lichtstrahls S1 angesteuert wird, und danach wird nur der zweite Laser L2 angesteuert, zum Ausgeben eines zweiten Lichtstrahls S2.

Fig. 6 zeigt ein schematisches Blockschaltbild eines Gehäuses 101 umfassend eine Sensorvorrichtung 1 nach einer der Fig. 1, 4 oder 5 sowie einen Handgriff 102.

Das Gehäuse 101 ist so gestaltet, dass die darin untergebrachte Sensorvorrichtung 1 mobil eingesetzt werden kann.

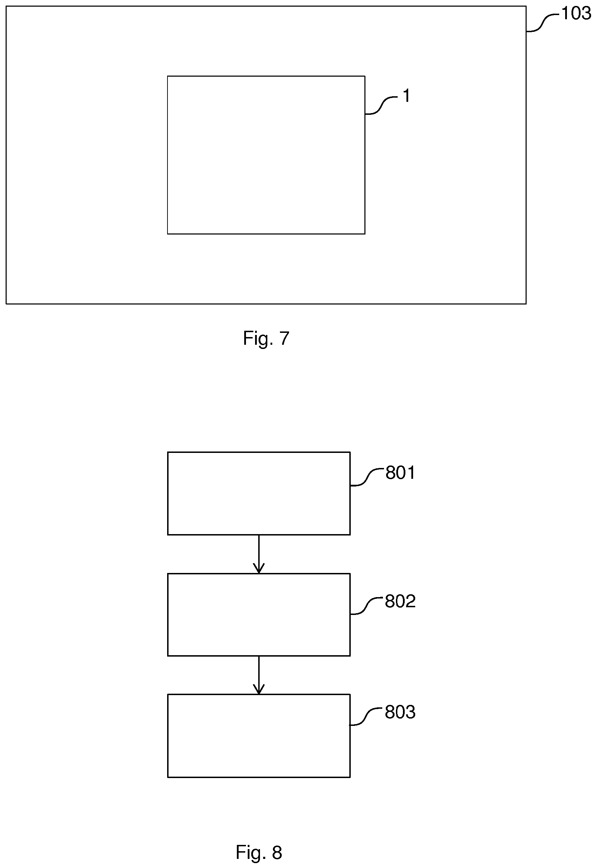

Fig. 7 zeigt ein schematisches Blockschaltbild eines wasserführenden Haushaltsgeräts 103 mit einer Sensorvorrichtung 1 nach einer der Fig. 1, 4 oder 5. Das wasserführende Haushaltsgerät 103 ist beispielsweise eine Geschirrspülmaschine, eine Waschmaschine, ein Wäschetrockner, eine Kaffeemaschine, ein Wasserkocher und/oder ein Dampfgarer.

Fig. 8 zeigt ein Verfahren zum Betreiben der Sensorvorrichtung 1. Das Verfahren umfasst dabei die Verfahrensschritte 801, 802 und 803.

In Verfahrensschritt 801 wird zumindest ein Lichtstrahl S1, S2, welcher eine vorbestimmte Strahlcharakteristik mit einer bestimmten Lichtintensität I1 aufweist, abgestrahlt.

In Verfahrensschritt 802 wird ein Behälter 3 zum Aufnehmen eines Gemisches 2 aus einer Flüssigkeit und einem Indikator T mittels des zumindest einen Lichtstrahls S1, S2 durchstrahlt.

In Verfahrensschritt 803 wird eine durch den mit dem Gemisch 2 befüllten Behälter 3 transmittierte Lichtintensität I2 des zumindest einem abgestrahlten Lichtstrahls S1, S2 detektiert.

Verwendete Bezugszeichen:

| 1 | Sensorvorrichtung |
|---|---|
| 2 | Gemisch |
| 3 | Behälter |
| 4 | Detektor |
| 5 | Bestimmungseinheit |
| 6 | Detektionsfläche |
| 7 | Strahlquerschnitt |
| 8 | Seitenwände |
| 9 | Mischvorrichtung |
| 10 | weiterer Behälter |
| 11 | Zuführvorrichtung |
| 12 | Steuerungseinrichtung |
| 13 | Speichermedium |
| 14 | Ausgabevorrichtung |
| 15 | Zusammenführungsoptik |

8

16 Fokussieroptik
17 Leitungswasserzuführung
18 Auslassleitung
101 Gehäuse
102 Handgriff
103 wasserführendes Haushaltsgerät
801 Verfahrensschritt
802 Verfahrensschritt
803 Verfahrensschritt

I1 bestimmte Lichtintensität
T Indikator
I2 transmittierte Lichtintensität
L1 Laser 1
L2 Laser 2
S1 Lichtstrahl 1
S2 Lichtstrahl 2

**Patentansprüche**

1. Wasserführendes Haushaltsgerät (103), insbesondere Geschirrspülmaschine, mit einer Sensorvorrichtung (1) zum Bestimmen zumindest einer Eigenschaft einer Flüssigkeit, die das dem wasserführenden Haushaltsgerät zugeführte Leitungswasser ist, wobei die Sensorvorrichtung (1) umfasst:

   einen ersten Laser (L1) und einen zweiten Laser (L2), wobei
   der erste Laser (L1) zum Abstrahlen eines ersten Lichtstrahls (S1), welcher eine erste vorbestimmte Strahlcharakteristik mit einer ersten bestimmten Lichtintensität (I1) aufweist, eingerichtet ist,
   der zweite Laser (L2) zum Abstrahlen eines zweiten Lichtstrahls (S2), welcher eine zweite vorbestimmte Strahlcharakteristik mit einer zweiten bestimmten Lichtintensität (I1) aufweist, eingerichtet ist,
   zumindest einen vor dem ersten und dem zweiten Laser (L1, L2) angeordneten Behälter (3), der mit einem Gemisch (2) aus der Flüssigkeit und zumindest einem Indikator (T) befüllt ist,
   zumindest einen Detektor (4) zum Detektieren einer durch den mit dem Gemisch (2) befüllten Behälter (3) transmittierten Lichtintensität (I2) des von dem ersten Laser (L1) abgestrahlten ersten Lichtstrahls (S1) und zum Detektieren einer durch den mit dem Gemisch (2) befüllten Behälters (3) transmittierten zweiten Lichtintensität (I2) des von dem zweiten Laser (L2) abgestrahlten zweiten Lichtstrahls (S2), und
   zumindest einen weiteren Behälter (10) zum Aufnehmen des zumindest einen Indikators (T) und zumindest eine zwischen dem zumindest einen Behälter (3) und dem zumindest einen weiteren Behälter (10) angeordnete, steuerbare Zuführvorrichtung (11) zum Zuführen des zumindest einen Indikators (T) aus dem zumindest einen weiteren Behälter (10) in den zumindest einen Behälter (3), wobei der zumindest eine Indikator (T) einen ersten Indikator (T1) und einen zweiten Indikator (T2) umfasst, und wobei die Sensorvorrichtung (1) einen Wasserhärtesensor zum Bestimmen einer Härte der Flüssigkeit umfasst, wobei der erste Indikator (T1) dazu geeignet ist, mittels

einer chemischen Reaktion mit in der Flüssigkeit enthaltenen Calciumionen und/oder Magnesiumionen ein lösliches Produkt zu formen, welches bei einer Emissionswellenlänge des ersten Lasers (L1) eine Absorption in Abhängigkeit der Konzentration der in der Flüssigkeit enthaltenen Calciumionen und/oder Magnesiumionen aufweist, jedoch keine Absorption bei einer Emissionswellenlänge des zweiten Lasers (L2) aufweist, und

die Sensorvorrichtung (1) zusätzlich einen pH-Sensor zum Bestimmen eines pH-Wertes der Flüssigkeit umfasst, wobei der zweite Indikator (T2) dazu geeignet ist, mittels einer chemischen Reaktion in Abhängigkeit des pH-Wertes der Flüssigkeit ein lösliches Produkt zu formen, welches bei einer Emissionswellenlänge des zweiten Lasers (L2) eine Absorption aufweist, jedoch keine Absorption bei einer Emissionswellenlänge des ersten Lasers (L1) aufweist, und wobei die Sensorvorrichtung (1) eine Bestimmungseinheit (5) aufweist, welche dazu eingerichtet ist, die Härte und den pH-Wert der Flüssigkeit in Abhängigkeit von den detektierten Lichtintensitäten zu bestimmen.

2. Wasserführendes Haushaltsgerät nach Anspruch 1, **dadurch gekennzeichnet, dass** eine Detektionsfläche (6) des Detektors (4) der Sensorvorrichtung (1) derart dimensioniert ist, dass der gesamte Strahlquerschnitt (7) des ersten und zweiten Lichtstrahls (S1, S2) durch den Detektor (4) detektierbar ist.

3. Wasserführendes Haushaltsgerät nach Anspruch 1 oder 2, **dadurch gekennzeichnet, dass** der Behälter (3) der Sensorvorrichtung (1) ein Kunststoffbehälter oder ein Glasbehälter ist, wobei zumindest zwei Seitenwände (8) in zu dem ersten und zweiten Lichtstrahl (S1, S2) normaler Richtung planparallel ausgeführt sind und in einem Bereich einer Emissionswellenlänge des ersten und zweiten Lasers (L1, L2) transparent sind.

4. Wasserführendes Haushaltsgerät nach einem der Ansprüche 1 bis 3, **dadurch gekennzeichnet, dass** der zumindest eine Behälter (3) der Sensorvorrichtung (1) eine Mischvorrichtung (9) zum Durchmischen des Gemisches (2) umfasst.

**5.** Wasserführendes Haushaltsgerät nach einem der Ansprüche 1 bis 4, **dadurch gekennzeichnet, dass** der zumindest eine Detektor (4) der Sensorvorrichtung (1) eine Fotodiode umfasst, wobei die Fotodiode eine Empfindlichkeit bei einer Emissionswellenlänge des ersten und zweiten Lasers (L1, L2) aufweist.

**6.** Wasserführendes Haushaltsgerät nach einem der Ansprüche 1 bis 5, **dadurch gekennzeichnet, dass** ein einziger vor dem ersten Laser (L1) und dem zweiten Laser (L2) angeordneter Behälter (3) zum Aufnehmen des Gemisches (2), und ein einziger Detektor (4) zum Detektieren einer durch den mit dem Gemisch (2) befüllten Behälter (3) transmittierten ersten Lichtintensität (l2) des von dem ersten Laser (L1) abgestrahlten ersten Lichtstrahls (S1) und zum Detektieren einer durch den mit dem Gemisch (2) befüllten Behälters (3) transmittierten zweiten Lichtintensität (l2) des von dem zweiten Laser (L2) abgestrahlten zweiten Lichtstrahls (S2) vorgesehen ist.

**7.** Wasserführendes Haushaltsgerät nach einem der Ansprüche 1 bis 6, **dadurch gekennzeichnet, dass** die Sensorvorrichtung (1) eine Steuerungseinrichtung (12) aufweist, welche dazu eingerichtet ist, das Füllen des Behälters (3) mit der Flüssigkeit, das Zuführen eines vorgegebenen Anteils des zumindest einen Indikators (T) über die zumindest eine Zuführvorrichtung (11) von dem zumindest einen weiteren Behälter (10) in den Behälter (3), das Durchmischen des Gemisches (2) durch die Mischvorrichtung (9), das Abstrahlen des ersten und zweiten Lichtstrahls (S1, S2) durch den ersten und zweiten Laser (L1, L2), das Detektieren der durch den Behälter (3) transmittierten ersten und zweiten Lichtintensität des ersten und zweiten Lichtstrahls (S1, S2) durch den Detektor (4), das Bestimmen der zumindest einen Eigenschaft der Flüssigkeit in Abhängigkeit von der transmittierten ersten und zweiten Lichtintensität (l2) durch die Bestimmungseinheit (5), und ein Speichern der zumindest einen Eigenschaft der Flüssigkeit auf einem Speichermedium (13) und/oder ein Ausgeben der zumindest einen Eigenschaft der Flüssigkeit über eine Ausgabevorrichtung (14) zu veranlassen.

**Claims**

**1.** Water-carrying household appliance (103), in particular dishwasher, with a sensor apparatus (1) for determining at least one property of a liquid, which is the mains water supplied to the water-carrying household appliance, wherein the sensor apparatus (1) comprises:

a first laser (L1) and a second laser (L2), wherein the first laser (L1) is designed to emit a first light beam (S1), which has a first predetermined beam characteristic with a first determined light intensity (11),

the second laser (L2) is designed to emit a second light beam (S2) which has a second predetermined beam characteristic with a second determined light intensity (11),

at least one container (3) arranged in front of the first and the second laser (L1, L2), which is filled with a mixture (2) of the liquid and at least one indicator (T),

at least one detector (4) for detecting a light intensity (12), transmitted through the container (3) filled with the mixture (2), of the first light beam (S1) emitted by the first laser (L1) and for detecting a second light intensity (2), transmitted through the container (3) filled with the mixture (2), of the second light beam (S2) emitted by the second laser (L2), and

at least one further container (10) for receiving the at least one indicator (T) and at least one controllable supply apparatus (11) arranged between the at least one container (3) and the at least one further container (10) for supplying the at least one indicator (T) from the at least one further container (10) into the at least one container (3), wherein

the at least one indicator (T) comprises a first indicator (T1) and a second indicator (T2), and wherein

the sensor apparatus (1) comprises a water hardness sensor for determining a hardness of the liquid, wherein the first indicator (T1) is suited to forming a soluble product by means of a chemical reaction with calcium ions and/or magnesium ions contained in the liquid, which, in the case of an emission wavelength of the first laser (L1), has an absorption as a function of the concentration of the calcium ions and/or magnesium ions contained in the liquid, but has no absorption in the case of an emission wavelength of the second laser (L2), and

the sensor apparatus (1) additionally comprises a pH sensor for determining a pH value of the liquid, wherein the second indicator (T2) is suited to forming a soluble product by means of a chemical reaction as a function of the pH value of the liquid, which in the case of an emission wavelength of the second laser (L2) has an absorption but in the case of an emission wavelength of the first laser (L1) has no absorption, and wherein the sensor apparatus (1) has a determination unit (5), which is designed to determine the hardness and the pH value of the liquid as a function of the detected light intensities.

**2.** Water-carrying household appliance according to claim 1, **characterised in that** a detection surface (6) of the detector (4) of the sensor apparatus (1) is dimensioned so that the entire beam cross-section (7) of the first and second light beam (S1, S2) can be detected by the detector (4).

**3.** Water-carrying household appliance according to claim 1 or 2, **characterised in that** the container (3) of the sensor apparatus (1) is a plastic container or a glass container, wherein at least two side walls (8) are configured to be plane-parallel in the normal direction with respect to the first and second light beam (S1, S2) and are transparent in a region of an emission wavelength of the first and second laser (L1, L2).

**4.** Water-carrying household appliance according to one of claims 1 to 3, **characterised in that** the at least one container (3) of the sensor apparatus (1) comprises a mixing apparatus (9) for stirring the mixture (2).

**5.** Water-carrying household appliance according to one of claims 1 to 4, **characterised in that** the at least one detector (4) of the sensor apparatus (1) comprises a photodiode, wherein the photodiode has a sensitivity in the case of an emission wavelength of the first and second laser (L1, L2).

**6.** Water-carrying household appliance according to one of claims 1 to 5, **characterised in that** a single container (3) arranged in front of the first laser (L1) and the second laser (L2) for receiving the mixture (2), and a single detector (4) for detecting a first light intensity (12), transmitted through the container (3) filled with the mixture (2), of the first light beam (S1) emitted by the first laser (L1) and for detecting a second light intensity (12), transmitted through the container (3) filled with the mixture (2), of the second light beam (S2) emitted by the second laser (L2), is provided.

**7.** Water-carrying household appliance according to one of claims 1 to 6, **characterised in that** the sensor apparatus (1) has a control facility (12), which is designed to trigger the filling of the container (3) with the liquid, the supply of a predetermined portion of the at least one indicator (T) by way of the at least one supply apparatus (11) from the at least one further container (10) into the container (3), the stirring of the mixture (2) by the mixing apparatus (9), the emission of the first and second light beam (S1, S2) by the first and second laser (L1, L2), the detection of the first and second light intensity, transmitted through the container (3), of the first and second light beam (S1, S2) by the detector (4), the determination of the at least one property of the liquid as a function of the transmitted first and second light intensity (12) by the determination unit (5), and a storage of the at least one property of the liquid on a storage medium (13) and/or an outputting of the at least one property of the liquid by way of an output apparatus (14).

## Revendications

**1.** Appareil ménager à circulation d'eau (103), en particulier lave-vaisselle, avec un dispositif de capteurs (1) pour la détermination d'au moins une propriété d'un fluide qui est l'eau de distribution dont l'appareil ménager à circulation d'eau est alimenté, dans lequel le dispositif de capteurs (1) comprend: un premier laser (L1) et un deuxième laser (L2), dans lequel le premier laser (L1) est aménagé afin d'émettre un premier rayon lumineux (S1), lequel présente une première caractéristique de rayon déterminée au préalable avec une première intensité lumineuse déterminée (I1), le deuxième laser (L2) est aménagé afin d'émettre un deuxième rayon lumineux (S2), lequel présente une deuxième caractéristique de rayon déterminée au préalable avec une deuxième intensité lumineuse déterminée (I1), au moins un contenant (3) disposé avant le premier et le deuxième laser (L1, L2), lequel contenant est rempli d'un mélange (2) issu du fluide et d'au moins un indicateur (T), au moins un détecteur (4) pour la détection d'une intensité lumineuse (I2) du premier rayon lumineux (S1) émis par le premier laser (L1) transmise à travers le contenant (3) rempli du mélange (2) et pour la détection d'une deuxième intensité lumineuse (I2) du deuxième rayon lumineux (S2) émis par le deuxième laser (L2) transmise à travers le contenant (3) rempli du mélange (2), et au moins un contenant supplémentaire (10) pour l'accueil de l'au moins un indicateur (T) et au moins un dispositif d'alimentation commandable (11) disposé entre l'au moins un contenant (3) et l'au moins un contenant supplémentaire (10) pour l'alimentation de l'au moins un indicateur (T) au départ de l'au moins un contenant supplémentaire (10) dans l'au moins un contenant (3), dans lequel l'au moins un indicateur (T) comprend un premier indicateur (T1) et un deuxième indicateur (T2), et dans lequel le dispositif de capteurs (1) comprend un capteur de dureté d'eau pour la détermination d'une dureté du fluide, dans lequel le premier indicateur (T1) est approprié afin de former, au moyen d'une réaction chimique avec les ions de calcium et/ou les ions de magnésium contenus dans le fluide, un produit soluble présentant en présence d'une longueur d'ondes d'émission du premier laser (L1) une absorption en fonction de la concentration des ions de calcium et/ou des ions de magnésium contenus dans le fluide, ne présentant cependant aucune absorption en présence d'une longueur d'ondes d'émission du deuxième laser (L2), et le dispositif de capteurs (1) présente en outre un capteur de pH pour la détermination d'une valeur pH du fluide, dans lequel le deuxième indicateur (T2) est approprié afin de former, au moyen d'une réaction chimique en fonction de la valeur pH du fluide, un produit soluble présentant en présence d'une longueur d'ondes d'émission du deuxième laser (L2) une absorption, ne présentant cependant aucune absorption en présence d'une longueur d'ondes d'émission du premier laser (L1), et dans lequel le dispositif de capteurs (1) présente une unité de détermination (5) aménagée afin de déterminer la dureté et la valeur pH du fluide en fonction des intensités lumineuses détectées.

2. Appareil ménager à circulation d'eau selon la revendication 1, **caractérisé en ce qu'**une surface de détection (6) du détecteur (4) du dispositif de capteurs (1) est dimensionnée de telle sorte que l'ensemble de la section de rayon (7) du premier et deuxième rayon lumineux (S1, S2) est détectable par le détecteur (4).

3. Appareil ménager à circulation d'eau selon la revendication 1 ou 2, **caractérisé en ce que** le contenant (3) du dispositif de capteurs (1) est un contenant en plastique ou un contenant en verre, dans lequel au moins deux parois latérales (8) sont exécutées, en direction normale vers le premier et le deuxième rayon lumineux (S1, S2), dans des plans parallèles et sont transparentes dans une plage d'une longueur d'ondes d'émission du premier et du deuxième laser (L1, L2).

4. Appareil ménager à circulation d'eau selon l'une des revendications 1 à 3, **caractérisé en ce que** l'au moins un contenant (3) du dispositif de capteurs (1) comprend un dispositif de mélange (9) pour le mélange du mélange (2).

5. Appareil ménager à circulation d'eau selon l'une des revendications 1 à 4, **caractérisé en ce que** l'au moins un détecteur (4) du dispositif de capteurs (1) comprend une photodiode, dans lequel la photodiode présente une sensibilité en présence d'une longueur d'ondes d'émission du premier et deuxième laser (L1, L2).

6. Appareil ménager à circulation d'eau selon l'une des revendications 1 à 5, **caractérisé en ce qu'**un seul contenant (3) disposé avant le premier laser (L1) et le deuxième laser (L2) est prévu pour l'accueil du mélange (2) et un seul détecteur (4) est prévu pour la détection d'une première intensité lumineuse (I2) du premier rayon lumineux (S1) émis par le premier laser (L1) transmise à travers le contenant (3) rempli du mélange (2) et pour la détection d'une deuxième intensité lumineuse (I2) du deuxième rayon lumineux (S2) émis par le deuxième laser (L2) transmise à travers le contenant (3) rempli du mélange (2).

7. Appareil ménager à circulation d'eau selon l'une des revendications 1 à 6, **caractérisé en ce que** le dispositif de capteurs (1) présente un dispositif de commande (12) aménagé afin d'ordonner le remplissage du contenant (3) en fluide, l'alimentation d'une quantité prescrite de l'au moins un indicateur (T) via l'au moins un dispositif d'alimentation (11) de l'au moins un contenant supplémentaire (10) dans le contenant (3), le mélange du mélange (2) par le dispositif de mélange (9), l'émission du premier et deuxième rayon lumineux (S1, S2) par le premier et deuxième laser (L1, L2), la détection par le détecteur (4) de la première et deuxième intensité lumineuse du premier et deuxième rayon lumineux (S1, S2) transmise à travers le contenant (3), la détermination par l'unité de détermination (5) de l'au moins une caractéristique du fluide en fonction de la première et deuxième intensité lumineuse (I2) transmise et un enregistrement de l'au moins une caractéristique du fluide sur un support d'enregistrement (13) et/ou une émission de l'au moins une caractéristique du fluide via un dispositif d'émission (14).

Fig. 1

Fig. 2

Fig. 3

Fig. 4

Fig. 5

Fig. 6

103

1

Fig. 7

801

802

803

Fig. 8

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 8147758 B2
- US 3652861 A
- US 3729263 A
- DE 102012011195 A1
- DE 102004015387 A1
- US 20070178595 A1
- DE 102009029305 A1
- DE 2013114011 A1
- JP 2001201497 A